# EUROPÄISCHE PATENTANMELDUNG

(11) **EP 2 702 936 A1**
(43) Veröffentlichungstag der Anmeldung: **05.03.2014**
(21) Anmeldenummer: 13179537.9
(22) Anmeldetag: 07.08.2013
(51) Int. Cl.: A61B 5/00, G02B 25/02

(54) **Abstandsaufsatz für ein Dermatoskop**

(30) Priorität: 04.09.2012 DE 202012008449 U
(71) Anmelder: Heine Optotechnik GmbH & Co KG, 82211 Herrsching (DE)
(72) Erfinder: Heine, Oliver, 82211 Herrsching (DE); Behrendt, Wolfgang, 82229 Seefeld (DE); Raab, Roman, 82211 Herrsching (DE)
(74) Vertreter: Hano, Christian

(57) **Zusammenfassung**

Der Abstandsaufsatz für ein Dermatoskop umfasst eine Außenhülse (12), deren eines Befestigungsende zur lösbaren Befestigung an dem Beleuchtungskopf eines Dermatoskops ausgebildet ist und deren anderes Kontaktende zur Auflage auf die Haut eines Patienten an einem Untersuchungsort dient. In der Außenhülse (12) ist ein Filtereinsatz (30) durch Drehung um die Mittelachse der Außenhülse (12) lösbar befestigt, der eine Beobachtungsscheibe (52) aufweist, durch die die Mittelachse der Außenhülse (12) senkrecht hindurchgeht. Der Filtereinsatz (30) ist mittels einer Drehschnappeinrichtung an der Außenhülse (12) lösbar befestigt, die wenigstens einen an dem Außenumfang des Filtereinsatzes (30) angeordneten elastischen Schnapphaken (44) umfasst, der einen an der Innenwandung der Außenhülse (12) vorgesehenen Vorsprung (26) elastisch umgreift.

## Beschreibung

Die Erfindung betrifft einen Abstandsaufsatz für ein Dermatoskop nach dem Oberbegriff des Patentanspruchs 1.

Dermatoskope umfassen eine vergrößernde Optik, eine Beleuchtung, die den Untersuchungsbereich möglichst ohne Reflexionen ausleuchtet, sowie eine Stromversorgung, die die Beleuchtung mit elektrischer Energie versorgt. Bei der Untersuchung wird das Dermatoskop normalerweise mit einer Kontaktscheibe aus Glas auf die Haut aufgesetzt und durch die Optik betrachtet. Bei bestimmten Ausführungsformen wird zwischen Dermatoskop bzw. der Kontaktscheibe und der Haut ein Dermatoskopieöl oder eine andere Flüssigkeit mit einem glasähnlichen Brechungsindex eingebracht. Bei anderen Ausführungsformen wird eine polarisierte Beleuchtung verwendet, so dass eine Kontaktscheibe und das Einbringen von Dermatoskopieöl nicht erforderlich sind. Einige Diagnosen sind nur bei Betrachtung mit polarisierter Beleuchtung möglich, während andere nur ohne polarisierte Beleuchtung durchgeführt werden können. Deshalb ist es von Vorteil, wenn das Dermatoskop mit beiden Arten von Beleuchtungsverfahren betrieben werden kann. Bei der Entwicklung eines entsprechenden Geräts wird daher darauf Wert gelegt, dass ohne großen Aufwand beide Beleuchtungsverfahren angewendet werden können.

Aus der US 7 167 243 B2 ist ein Dermatoskop bekannt, mit dem beide Beleuchtungsverfahren durchgeführt werden können. Eine Vielzahl von Leuchtdioden sind in einem Kreisring angeordnet, vor dem sich ein ringförmiger Polarisationsfilter befindet, der an einigen Stellen ausgespart ist. Einige Leuchtdioden sind in ihrer Beleuchtungsrichtung gesehen vor den ausgesparten Stellen angeordnet und andere vor dem Polarisationsfilter. Durch eine elektronische Schaltung kann mittels eines Schalters entweder die polarisierte Beleuchtung oder die unpolarisierte Beleuchtung geschaltet werden, so dass beide Beleuchtungsverfahren möglich sind. Der Polarisationsfilter, der in der Beobachtungsachse angeordnet ist, ist nicht auswechselbar, so dass nur bestimmte Diagnosen möglich sind. Ein weiterer Nachteil des Polarisationsfilters in der Beobachtungsachse ist es, dass dieser eine dämpfende Wirkung von rund 40% auf die Helligkeit in der Beobachtung hat. Viele Polarisationsfilter sind zudem nicht farbneutral, so dass zwar eine Diagnose ohne Polarisierung grundsätzlich möglich ist, aber der Polarisationsfilter einen unnatürlichen Farbstich erzeugt, der die Diagnose beeinträchtigen könnte.

Aus der DE 200 22 603 U1 ist ein Dermatoskop mit einem gattungsgemäßen Abstandaufsatz bekannt. Das Dermatoskop weist einen Griffteil auf, in den die Stromversorgung für eine Beleuchtungseinrichtung angebracht ist. An dem Griffteil ist ein Beleuchtungskopf vorgesehen, durch den ein Beobachtungskanal hindurchgeht, in dem eine Lupe angeordnet ist. An der unteren Stirnseite des Beleuchtungskopfes sind mehrere Leuchtdioden in gleichmäßigem Umfangsabstand um den Beleuchtungskanal vorgesehen. Außerdem ist an der unteren Stirnseite des Beleuchtungskopfes ein Abstandsaufsatz angebracht, der an seinem unteren Ende ein Kontaktglas aufweist, das bei einer Diagnose auf die Haut eines Patienten aufgesetzt wird.

Der Erfindung liegt die Aufgabe zugrunde, mit konstruktiv einfachen Mitteln einen Abstandsaufsatz für ein Dermatoskop zu schaffen, der es ermöglicht, mit wenig Aufwand das Dermatoskop für viele unterschiedliche Diagnosen optimal anzupassen.

Diese Aufgabe wird erfindungsgemäß durch einen Abstandsaufsatz für ein Dermatoskop mit den Merkmalen des Patentanspruchs 1 gelöst. Vorteilhafte Weiterbildungen des erfindungsgemäßen Abstandsaufsatzes sind Gegenstand der Patentansprüche 2 bis 6.

Bei dem erfindungsgemäßen Abstandsaufsatz ist in seiner Außenhülse ein Filtereinsatz durch Drehung um die Mittelachse der Außenhülse lösbar befestigt, der eine Beobachtungsscheibe aufweist, durch die die Mittelachse der Außenhülse senkrecht hindurchgeht. Auf diese Weise kann der Filtereinsatz einfach durch Drehung um die Mittelachse von der Außenhülse gelöst und durch einen neuen Filtereinsatz ersetzt werden, der für eine neue Diagnose vorteilhaft ist.

Der Filtereinsatz ist mittels einer Drehschnappeinrichtung an der Außenhülse lösbar befestigt, die wenigstens einen an dem Außenumfang des Filtereinsatzes angeordneten elastischen Schnapphaken umfasst, der einen an der Innenwandung der Außenhülse vorgesehenen Vorsprung elastisch umgreift.

Für eine optimale Beleuchtung umfasst der Filtereinsatz einen die Beobachtungsscheibe umgebenden Fensterring, in dem mehrere lichtdurchlässige Beleuchtungsfenster angeordnet sind. Wenn der Abstandsaufsatz an einem entsprechenden Dermatoskop angebracht wird, sind die lichtdurchlässigen Beleuchtungsfenster jeweils vor einem Leuchtmittel, bevorzugt einer LED angeordnet.

In den Beleuchtungsfenstern kann ein Beleuchtungs-Polfilter angeordnet und in der Beobachtungsscheibe ein Beobachtungs-Polfilter angeordnet sein. Die Beleuchtungs-Polfilter sind bei einer bevorzugten Ausführungsform bezüglich des Beobachtungs-Polfilters kreuzpolarisiert.

Das Kontaktende der Außenhülse kann durch eine transparente Kontaktscheibe verschlossen sein, wie es bei dem aus der DE 200 22 603 U1 bekannten Dermatoskop der Fall ist.

Um die verschiedensten Diagnosen durchzuführen, ist ein Kit für einen Abstandsaufsatz zweckmäßig, das mehrere gegenseitig austauschbare Filtereinsätze aufweist. Die Filtereinsätze können beispielsweise mit einen Linear-Polfilter und/oder einem Neutraldichtefilter und/oder einem Zirkular-Polfilter und/oder einem Bandpassfilter und /oder einem Fluoreszenzfilter versehen werden.

Ein Dermatoskop mit einem erfindungsgemäßen Abstandsaufsatz auf seiner Beleuchtungsseite ist Gegenstand des Patentanspruchs 9. An der Beleuchtungsseite des Beleuchtungskopfs sind bevorzugt mehrere Leuchtmittel, vorzugsweise LEDs, jeweils in Beleuchtungsrichtung des Leuchtmittels gesehen vor einem Fenster des Filtereinsatzes angeordnet.

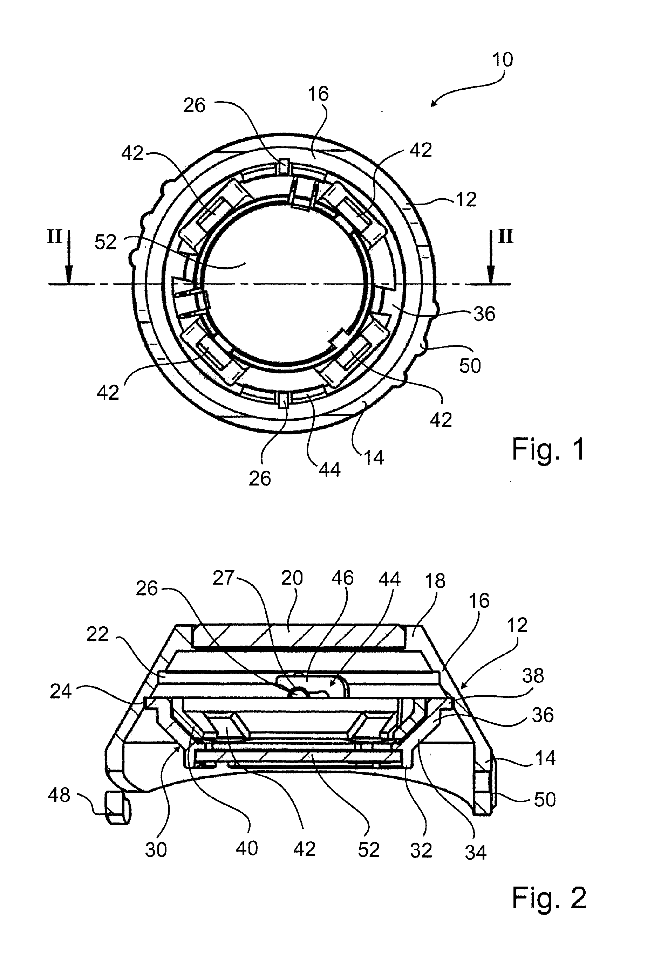

Ein Ausführungsbeispiel der Erfindung wird nachstehend anhand von Zeichnungen näher erläutert. Es zeigen
Fig. 1 eine Unteransicht eines Abstandsaufsatzes,
Fig. 2 den Schnitt II-II von Figur 1,
Fig. 3 im Querschnitt ein Dermatoskop im Bereich eines Beleuchtungskopfes, an dem der Abstandsaufsatz von Fig. 1 angebracht ist.

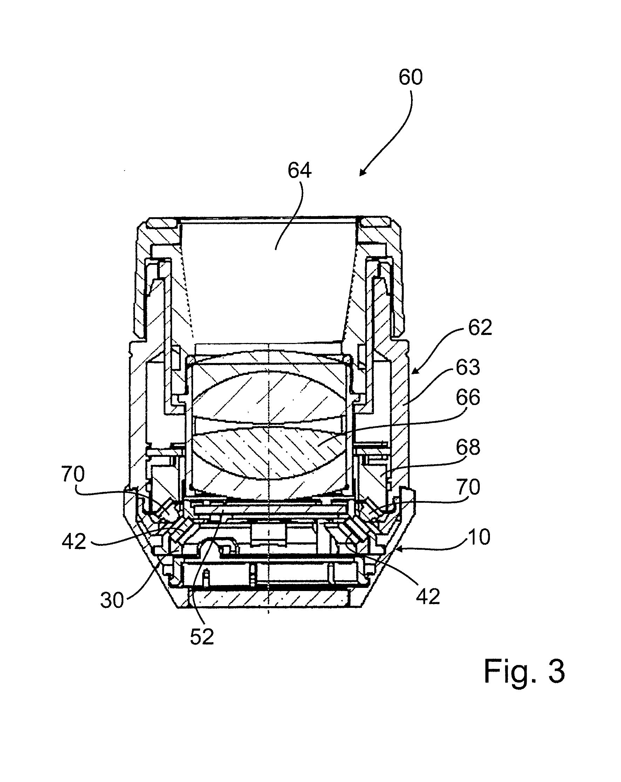

Der Abstandsaufsatz 10 umfasst eine im Wesentlichen rotationssymmetrische Außenhülse 12 mit einem der Beleuchtungsseite eines Dermatoskops 60 (Fig. 3) zugewandten, im Wesentlichen zylindrischen unteren Abschnitt 14, an den ein sich konisch nach oben verjüngender Konusabschnitt 16 anschließt. In die obere Stirnseite der Außenhülse 12 ist eine lichtdurchlässige Kontaktscheibe 20 eingesetzt.

An der Innenwandung des konischen Abschnitts 16 der Außenhülse 12 ist eine untere zylindrische Ringaussparung 24 ausgebildet, wodurch eine Stufe gebildet wird. Eine weitere zylindrische Ringaussparung 22 befindet sich zwischen der Kontaktscheibe 20 und der Ringaussparung 24.

An der Außenseite des zylindrischen Abschnitts 14 der Außenhülse 12 sind Rippen 50 vorgesehen, um eine Drehung der Außenhülse 12 für die Befestigung an einem Beleuchtungskopf eines Dermatoskops zu erleichtern. Außerdem ist ein sich in Umfangsrichtung erstreckender Rasthaken 48 vorgesehen, der bei der Drehbefestigung der Außenhülse 12 an dem Beleuchtungskopf in eine entsprechende Aussparung an dem Beleuchtungskopf schnappend eingreift.

In die Außenhülse 12 ist ein im Wesentlichen rotationssymmetrischer Filtereinsatz 30 eingesetzt. Der Filtereinsatz 30 weist einen oberen Ringabschnitt 38 auf, der in die zylindrische Ringaussparung 24 eingreift. An den Ringabschnitt 38 schließt unten ein koaxialer zylindrischer Abschnitt 36 mit geringerem Durchmesser an, der in einen sich nach unten konisch verjüngenden Konusabschnitt 34 übergeht, an den ein weiterer zylindrischer Abschnitt 32 koaxial anschließt. In dem zylindrischen Abschnitt 32 ist eine Beobachtungsscheibe 52 gehalten. Innerhalb des Filtereinsatzes 30 ist ein ringförmiger Fenstereinsatz 40 vorgesehen, der im Bereich des Ringabschnitts 38 und des Konusabschnitts 34 an der Innenseite des Filtereinsatzes 30 anliegt. In dem Fenstereinsatz 40 sind im Bereich des Konusabschnitts 34 Fenster 42 ausgebildet, die jeweils vor einer LED in einem Beleuchtungskopf angeordnet sind, wenn der Abstandsaufsatz 10 an dem Beleuchtungskopf eines Dermatoskops befestigt ist. Der Konusabschnitt 34 ist an diesen Stellen entsprechend ausgespart.

Unterhalb der Ringaussparung 22 sind diametral gegenüberliegend zwei Rastzapfen 26 vorgesehen, die radial von der Innenseite der Außenhülse 16 vorstehen. An der Außenseite des Filtereinsatzes 30 sind zwei Schnapphaken 44 diametral gegenüberliegend angeordnet, die einen sich in Umfangsrichtung erstreckenden Schnapparm 46 aufweisen, der so angeordnet und elastisch ausgebildet ist, dass er sich bei Drehung des Filtereinsatzes 30 innerhalb der Außenhülse 12 gleitend über den jeweiligen Rastzapfen 26 bewegt und diesen dann elastisch übergreift, bis der Rastzapfen 26 in eine Rastmulde 27 in dem Schnapparm 46 eingreift. Der Filtereinsatz 30 wird dabei mit seinem Ringabschnitt 38 in der Ringaussparung 24 zum Anliegen an die Stufe gebracht und somit lagegerecht in der Außenhülse 12 fixiert. Der Schnapparm 46 bewegt sich bei der Drehbewegung innerhalb der Ringaussparung 22.

Zum Lösen des Filtereinsatzes 30 aus der Außenhülse 12 wird der Filtereinsatz 30 in die entgegengesetzte Richtung gedreht, so dass der Schnapphaken 44 den Rastzapfen 26 freigibt und der Filtereinsatz 30 anschließend aus der Außenhülse 12 gezogen werden kann.

In Fig. 3 ist ein Querschnitt eines Dermatoskops 60 im Bereich eines Beleuchtungskopfes 62 gezeigt. Der Beleuchtungskopf 62 umfasst ein insgesamt zylindrisches Gehäuse 63, in dessen obere Stirnseite ein Okular 64 eingesetzt ist. An das untere Ende des Gehäuses 63 ist der Abstandsaufsatz 10 aufgeschraubt. Zwischen dem unterem Ende des Okulars 64 und der Beobachtungsscheibe 52 des Abstandsaufsatzes 10 ist eine Abbildungsoptik 66 angeordnet, die an ihrem unteren Ende von einem Haltering 68 umgeben ist, in dessen unterer Stirnseite mehrere LEDs 70 in gleichmäßigem Umfangsabstand so befestigt sind, dass sie jeweils über einem Beleuchtungsfenster 42 angeordnet sind, d.h. in Beleuchtungsrichtung der LEDs 70 vor dem jeweiligen Beleuchtungsfenster 42.

Bei einer bevorzugten Ausführungsform sind in den Beleuchtungsfenstern 42 Beleuchtungs-Polfilter angeordnet, während die Beobachtungsscheibe 52 von einem Beobachtungs-Polfilter gebildet wird. Die beiden Polfilter sind kreuzpolarisiert, um Reflexionen zu eliminieren.

In den Beleuchtungsfenstern 42 können auch Neutraldichtefilter angeordnet sein und die Beobachtungsscheibe 52 kann aus hochwertigem Schutzglas gebildet sein. Mit dieser Lösung kann eine zu einem Polfiltereinsatz identische Helligkeit erzielt werden. Dies ist bei einer Diagnose wichtig, die einen Vergleich mit/ohne Polarisation notwendig macht.

Die Beobachtungsscheibe kann auch von einem zirkularen Polfilter gebildet werden ebenso wie die Beleuchtungsfenster. In diesem Fall ist eine Ausrichtung der Filter nicht notwendig.

Die Beleuchtungsfenster 42 und die Beobachtungsscheibe 52 können auch mit Bandpassfiltern versehen sein. Hierdurch kann der Kontrast bestimmter Farben erhöht werden, um damit sicher und schnell eine Diagnose für bestimmte Erkrankungen treffen zu können. Beispielsweise könnte als Bandpassfilter ein Grünfilter verwendet werden, der Rottöne besonders dunkel darstellt. Ebenso kann ein Rotfilter verwendet werden, der Grüntöne besonders dunkel kontrastiert.

In den Beleuchtungsfenstern 42 können auch Fluoreszenzfilter eingesetzt werden, die ein schmales Band von UV-Licht auf die Beobachtungsebene treffen lassen und Luminophore anregen und damit zum Leuchten bringen. Als Beobachtungsscheibe kann ein auf das Luminophor abgestimmter Filter verwendet werden, der das Signal-Rausch-Verhältnis und damit den Kontrast verbessert. Mit entsprechenden Kontrastmitteln könnte dann eine angiographische Untersuchung (Gefäßdarstellung, subkutane Blutgefäßdarstellung) durchgeführt werden.

## Patentansprüche

1. Abstandsaufsatz für ein Dermatoskop, mit einer Außenhülse (12), deren eines Befestigungsende zur lösbaren Befestigung an dem Beleuchtungskopf eines Dermatoskops ausgebildet ist und deren anderes Kontaktende zur Auflage auf die Haut eines Patienten an einem Untersuchungsort dient,
**dadurch gekennzeichnet, dass**
- in der Außenhülse (12) ein Filtereinsatz (30) durch Drehung um die Mittelachse der Außenhülse (12) lösbar befestigt ist, der eine Beobachtungsscheibe (52) aufweist, durch die die Mittelachse der Außenhülse (12) senkrecht hindurchgeht, und
- der Filtereinsatz (30) mittels einer Drehschnappeinrichtung an der Außenhülse (12) lösbar befestigt ist, die wenigstens einen an dem Außenumfang des Filtereinsatzes (30) angeordneten elastischen Schnapphaken (44) umfasst, der einen an der Innenwandung der Außenhülse (12) vorgesehenen Vorsprung (26) elastisch umgreift.

2. Abstandsaufsatz nach Anspruch 1, **dadurch gekennzeichnet, dass** der Filtereinsatz (30) einen die Beobachtungsscheibe (52) umgebenden Fensterring (40) umfasst, in dem mehrere lichtdurchlässige Beleuchtungsfenster (42) angeordnet sind.

3. Abstandsaufsatz nach Anspruch 1, **dadurch gekennzeichnet,**
**dass** in den Beleuchtungsfenstern (42) ein Beleuchtungs-Polfilter angeordnet ist.

4. Abstandsaufsatz nach einem der vorhergehenden Ansprüche, **dadurch gekennzeichnet,**
**dass** die Beobachtungsscheibe (52) ein Beobachtungs-Polfilter ist.

5. Abstandsaufsatz nach Anspruch 3 und 4, **dadurch gekennzeichnet,**
**dass** die Beleuchtungs-Polfilter bezüglich des Beobachtungs-Polfilters kreuzpolarisiert sind.

6. Abstandsaufsatz nach einem der vorhergehenden Ansprüche, **dadurch gekennzeichnet,**
**dass** das Kontaktende der Außenhülse (12) durch eine transparente Kontaktscheibe (20) verschlossen ist.

7. Kit für einen Abstandsaufsatz nach einem der Ansprüche 1 bis 6 mit mehreren gegenseitig austauschbaren Filtereinsätzen (30).

8. Kit nach Anspruch 7, **dadurch gekennzeichnet, dass** die Filtereinsätze (30) mit einem Linear-Polfilter und/oder einem Neutraldichtefilter und/oder einem Zirkular-Polfilter und/oder einem Bandpassfilter und /oder einem Fluoreszenzfilter versehen sind.

9. Dermatoskop mit einem Beleuchtungskopf, **dadurch gekennzeichnet, dass** ein Abstandsaufsatz (10) nach einem der Ansprüche 1 bis 6 an der Beleuchtungsseite des Beleuchtungskopfes angebracht ist.

10. Dermatoskop nach Anspruch 9 mit einem Abstandsaufsatz nach einem der Ansprüche 2 bis 6, **dadurch gekennzeichnet, dass** an der Beleuchtungsseite des Beleuchtungskopfs Leuchtmittel - in Beleuchtungsrichtung des Leuchtmittels gesehen - jeweils vor einem Beleuchtungsfenster (42) des Filtereinsatzes (30) angeordnet sind.

11. Dermatoskop nach Anspruch 10, **dadurch gekennzeichnet, dass** die Leuchtmittel LEDs sind.
